# EUROPEAN PATENT APPLICATION

(11) **EP 2 228 074 A1**
(43) Date of publication of application: **15.09.2010**
(21) Application number: 09155798.3
(22) Date of filing: 20.03.2009
(51) Int. Cl.: A61K 49/00, A61K 49/18, A61K 51/12, A61P 35/00, A61K 47/48

(54) **A tumor targeting protein conjugate and a method for preparing the same**

(30) Priority: 19.02.2009 KR 20090013745
(71) Applicant: Korea Institute of Science and Technology, Seoul 136-791 (KR)
(72) Inventor: Kwon, Ick Chan, 139-220, Seoul (KR); Kim, Kwang Meyung, 152-092, Seoul (KR); Choi, Kuiwon, 130-050, Seoul (KR)
(74) Representative: Hewson, Timothy John

(57) **Abstract**

The present invention relates to a protein conjugate having excellent tumor targeting capacity and a method for preparing the same, more precisely albumin-bile acid conjugate forming 30-500 nm nano-particles and forming self-aggregates in water system and a method for preparing the same. The albumin-bile acid conjugate of the present invention has excellent tumor targeting capacity and facilitates inclusion of a hydrophobic anticancer agent and can be combined with fore infrared ray fluorescent material. Therefore, this conjugate can be effectively used for the production of a novel nano-particle contrast agent or a nano-particle drug delivery system for the diagnosis and treatment of cancer.

## Description

This application claims the benefit of Korea Application No. 10-2009-0013745 filed on February 19, 2009, the contents of which are hereby incorporated by reference in their entirety.

### BACKGROUND OF THE INVENTION

### 1. Field of the Invention

The present invention relates to a protein conjugate having excellent tumor targeting capacity and a method for preparing the same, more precisely albumin-bile acid conjugate forming 30-500 nm nano-particles and forming self-aggregates in water system and a method for preparing the same.

### 2. Description of the Related Art

Albumin is a very stable protein that can be circulated at least for 20 days in blood. It is conjugated with the conventional low molecular chemicals, protein or antibody and then used as a drug delivery system to improve *in vivo* stability of diverse drugs. For example, the hydrophobic anticancer agents, doxorubicin (Cancer Research 53, 4238-4242, 1993) and methotrexate (MTX) (Anticancer Drugs 8, 835-844, 1997) have been chemically conjugated with albumin to improve tumor targeting capacity and to improve stability in blood.

Albumin based drug delivery system takes advantage of high tumor targeting capacity of albumin. Small animals having tumor tissues are image-processed by conjugating radio isotope or fluorescein to albumin. A large amount of albumin is accumulated selectively in tumor tissues at this time (Cancer Research 46, 6387-6392, 1986), which seems to be attributed to the enhanced permeability and retention (EPR) effect shown in loosened blood vessels around tumor tissues. It has been reported that when diverse anticancer agents, proteins or antibodies are chemically conjugated with albumin, circulation time in blood is extended, compared with the pure drug itself, and accumulation efficiency in tumor tissues is increased (Journal of Controlled Release, 132, 171-183, 2008).

However, when an anticancer agent is chemically conjugated with albumin, it might reduce anticancer effect or diverse anticancer agent derivatives can be generated because the separation mechanism between albumin and the conjugated anticancer agent is not disclosed, yet. To maintain properties of albumin, the number of anticancer agents for the chemical modification is limited to 1 - 3 per albumin molecule (Journal of Controlled Release, 132, 171-183, 2008).

To solve the above problem, American Bioscience developed a drug delivery system comprising nano-particles of 100-200 nm in size by physically stamping the hydrophobic anticancer agent Paclitaxel upon albumin by using jet stream which has been approved by FDA and being sold on market under the brand name of Abraxane for the treatment of metastatic breast cancer. The developed albumin nano-particle drug delivery system demonstrated excellent tumor targeting and improved treatment effect.

The present inventors continued to study to overcome side effects of the conventional drug delivery carriers using albumin. As a result, the inventors developed albumin nano-particles of 30 - 400 nm in size by chemically conjugating bile acid (5β-cholanic acid) with albumin. The developed albumin-bile acid conjugate is the nano-particle having 10-100 fold increased particle size, compared with the conventional albumin (2 ∼ 5 nm) and having significantly improved tumor targeting capacity and capable of forming self-aggregates. The present inventors completed this invention by confirming that it also has the advantage of micelle by physically harboring the hydrophobic anticancer agent in the self-aggregates, so that it can be used as a novel nano-particle drug delivery system and a nano-particle contrast agent for the diagnosis or treatment of cancer.

### SUMMARY OF THE INVENTION

It is an object of the present invention to provide albumin-bile acid conjugate having excellent tumor targeting capacity and facilitating hydrophobic anticancer inclusion based on albumin which is endogenous and has excellent biocompatibility, and an anticancer agent carrier and a contrast agent for cancer diagnosis using the same.

To achieve the above object, the present invention provides albumin-bile acid conjugate for drug delivery which is composed of hydrophilic albumin and hydrophobic bile acid so that it can form self-aggregates in aqueous solution.

The present invention also provides a method for preparing the said albumin-bile acid conjugate comprising the following steps:
1) preparing albumin solution by dissolving hydrophilic albumin in a water-soluble solvent;
2) preparing bile acid solution by dissolving hydrophobic bile acid in an organic solvent;
3) preparing reaction solution by mixing the albumin solution of step 1) and the bile acid solution of step 2) and stirring thereof; and
4) dialyzing the reaction solution of step 3) and eliminating non-reacted bile acid, followed by freeze-drying thereof.

The present invention further provides use of the said albumin-bile aid conjugate in the manufacture of a drug delivery carrier.

The present invention also provides a method for treating cancer comprising the following steps:
1) mixing an anticancer agent with the said albumin-bile acid conjugate, and forming an albumin-bile acid nanoparticle containing the anticancer agent thereby; and
2) administering the nanoparticle to a subject.

The present invention provides a contrast agent for cancer diagnosis comprising albumin-bile acid-fore infrared ray fluorescent material conjugate composed of the said albumin-bile acid conjugate and fore infrared ray fluorescent material.

The present invention also provides a method for diagnosing cancer comprising the following steps:
1) administering the said contrast agent to a subject; and
2) imaging of tumor tissues after irradiating fore infrared ray.

In addition, the present invention provides use of the said albumin-bile acid-fore infrared ray fluorescent material conjugate in the manufacture of a contrast agent for cancer diagnosis.

### ADVANTAGEOUS EFFECT

The albumin-bile acid conjugate of the present invention forms macro nano-particles, unlike pure albumin, and has high tumor selectivity based on EPR effect and excellent accumulation efficiency in tumor tissues. In addition, the conjugate has extended *in vivo* staying, compared with the conventional albumin, and forms self-aggregates in water-system, so that it favors inclusion of a hydrophobic anticancer agent, suggesting great possibility of being used as a drug delivery carrier. It can also be conjugated with fore infrared ray fluorescent material, which favors early diagnosis of cancer and long term imaging of tumor tissues and treatment of cancer.

### BRIEF DESCRIPTION OF THE DRAWINGS

The application of the preferred embodiments of the present invention is best understood with reference to the accompanying drawings, wherein:
Figure 1 is a diagram illustrating the method for preparing albumin-bile acid conjugate.
Figure 2 is a diagram showing the fore infrared ray fluorescence of albumin-bile acid-Cy5.5 conjugate detected by using fore infrared ray fluorospectrophotometer.
Figure 3 is a graph illustrating the changes of particle size of albumin-bile acid-Cy5.5 conjugate according to the increase of 5β-cholanic acid content in the albumin-bile acid-Cy5.5 conjugate.
Figure 4 is a diagram illustrating the shape of particle of albumin-bile acid-Cy5.5 conjugate in water-system, observed under transmission electron microscope (TEM).
Figure 5 is a diagram illustrating the drug accumulation efficiency in tumor tissues measured by fore infrared ray fluorospectrophotometer after intravenously injecting albumin-bile acid-CY5.5 conjugate into a mouse having tumor.

### DESCRIPTION OF THE PREFERRED EMBODIMENTS

Hereinafter, the present invention is described in detail.

The present invention provides albumin-bile acid conjugate for drug delivery comprising hydrophilic albumin and hydrophobic bile acid and forming self-aggregates in aqueous solution.

The said albumin-bile acid conjugate preferably has the structure in which carboxyl group (-COOH) of bile acid is linked to amino group (-NH2) of lysine group of albumin by peptide bond, but not always limited thereto.

The albumin-bile acid conjugate of the present invention preferably forms albumin-bile acid-fore infrared ray fluorescent material conjugate by binding additionally to fore infrared ray fluorescent material, but not always limited thereto.

The fore infrared ray fluorescent material herein is selected from the group consisting of cyanine (Cy), fluorescein, tetramethylrhodamine, BODIPY and Alexa, and more preferably it is Cy5.5, but not always limited thereto.

The albumin-bile acid-fore infrared ray fluorescent material conjugate of the present invention is preferably represented by the following structural formula, but not always limited thereto:

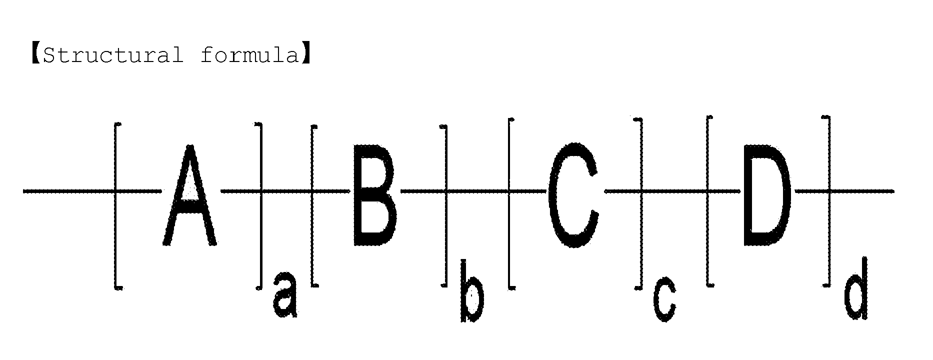

In the above formula, A is lysine group of albumin, B is bile acid conjugated with lysine group of albumin, C is fore infrared ray fluorescent material conjugated with lysine group of albumin, and D is an additional binding material for example radio isotope or gadolinium metal ion used as a contrast agent. And a indicates the number of lysine groups in albumin protein, ranging from 1 to 59, b indicates the number of bile acid conjugated lysine groups ranging from 1 to 59, c indicates the number of fore infrared ray fluorescent material conjugated lysine groups ranging from 1 to 10, and d indicates the number of additional binding materials conjugated thereto ranging from 0 to10.

Albumin herein is preferably human albumin, its fragment or its variant, but not always limited thereto. Albumin is endogenous *in vivo* and has excellent biocompatibility and excellent *in vivo* stability, suggesting high biodistribution in blood, so that it can be continuously accumulated in tumor tissues long enough. In particular, albumin contains lysine group a lot, so that it can be chemically conjugated with various anticancer agents, fluoresceins or radio-isotopes easily.

The said bile acid is a hydrophobic material which is preferably 5β-cholanic acid represented by formula 1, but not always limited thereto and any material selected from the group consisting of deoxycholic acid, taurodeoxycholic acid, taurocholic acid and glycochenodeoxyhoclic acid can be used.

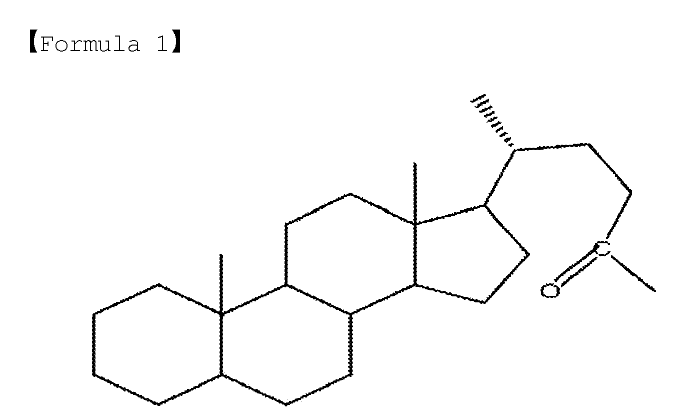

The albumin-bile acid conjugate of the present invention can form round shape self-aggregates similar to micelle in water-system owing to amphiphilicity resulted from hydrophilic group of albumin and hydrophobic group of bile acid.

Nano-particle of the albumin-bile acid conjugate of the present invention is preferably 20 nm - 500 nm in diameter, and more preferably 3 nm - 400 nm, but not always limited thereto. The particle size of albumin-bile acid conjugate depends on the amount of bile acid. Bile acid can be included in the conjugate at the concentration of 10 - 50 weight% by the amount of albumin.

The albumin-bile acid conjugate of the present invention can include a hydrophobic anticancer agent.

Inside of the albumin-bile acid conjugate of the present invention comprises hydrophobic bile acid and a part of hydrophilic region of bile acid is conjugated with albumin, so that the inner part of albumin-bile acid conjugate has strong hydrophobicity. So, a hydrophobic anticancer agent can be easily included in the albumin-bile acid conjugate, indicating an anticancer agent can be physically inserted in the inside of the albumin-bile acid conjugate. Since inner part of the albumin-bile acid conjugate and an anticancer agent to be inserted therein have hydrophobicity, the amount of a drug to be delivered can be increased, which is generally limited in the case of chemical binding, suggesting that this conjugate is effective in drug delivery, compared with other carriers.

An anticancer agent to be included physically in the inside of the albumin-bile acid conjugate of the present invention can be any hydrophobic anticancer agent selected from the group consisting of Docetaxel, cis-platin, camptothecin, paclitaxel, Tamoxifen, Anasterozole, Gleevec, 5-FU, Floxuridine, Leuprolide, Flutamide, Zoledronate, Doxorubicin, Vincristine, Gemcitabine, Streptozocin, Carboplatin, Topotecan, Belotecan, Irinotecan, Vinorelbine, hydroxyurea, Valrubicin, retinoic acid, Methotrexate, Meclorethamine, Chlorambucil, Busulfan, Doxifluridine, Vinblastin, Mitomycin, Prednisone, Testosterone, Mitoxantron, aspirin, salicylates, ibuprofen, naproxen, fenoprofen, indomethacin, phenyltazone, cyclophosphamide, mechlorethamine, dexamethasone, prednisolone, celecoxib, valdecoxib, nimesulide, cortisone and corticosteroid, but not always limited thereto.

To investigate inclusion efficiency of a hydrophobic anticancer agent in the inside of albumin-bile acid-Cy5.5 conjugate, albumin-bile acid-Cy5.5 conjugate solution and Taxsol solution were mixed and stirred, followed by centrifugation for quantification. As a result, the albumin-bile acid-Cy5.5 conjugate of the present invention demonstrated 30 - 50% of anticancer agent inclusion efficiency, and the anticancer agent inclusion efficiency was regulated by the concentration of bile acid.

The albumin-bile acid conjugate of the present invention has a significant tumor targeting capacity, compared with pure albumin.

To investigate accumulation of albumin-bile acid-Cy5.5 conjugate in tumor tissues, albumin and albumin-bile acid-Cy5.5 conjugate were intravenously injected into the mice transplanted with skin cancer cells. After a while, fore infrared ray irradiation was performed. As a result, the albumin-bile acid-Cy5.5 conjugate of the present invention demonstrated improved accumulation efficiency in tumor tissues, compared with pure albumin. And at this time, the accumulation efficiency was regulated by the concentration of bile acid.

Therefore, it was confirmed that the albumin-bile acid conjugate of the present invention had excellent biocompatibility, could form nano-particles easily in aqueous solution, and favored regulation of the size of nano-particle, efficiency of anticancer agent inclusion and efficiency of accumulation in tumor according to the concentration of the hydrophobic cholanic acid conjugated chemically to albumin. That is, it can be effectively used as a component for novel drug delivery system applicable in diagnosis or treatment of cancer because it has excellent hydrophobic anticancer agent inclusion efficiency and improved accumulation efficiency in tumor tissues.

The present invention also provides a method for preparing the said albumin-bile acid conjugate comprising the following steps:
1) preparing albumin solution by dissolving hydrophilic albumin in a water-soluble solvent;
2) preparing bile acid solution by dissolving hydrophobic bile acid in an organic solvent;
3) preparing reaction solution by mixing the albumin solution of step 1) and the bile acid solution of step 2) and stirring thereof; and
4) dialyzing the reaction solution of step 3) and eliminating non-reacted bile acid, followed by freeze-drying thereof.

In the above preparing method, the hydrophilic albumin of step 1) is preferably natural polymer water-soluble albumin having excellent biodegradability and biocompatibility, and more preferably human albumin, its fragment or its variant, but not always limited thereto. The water-soluble solvent can be any water-soluble solvent capable of dissolving hydrophilic albumin, and preferably water. The albumin herein is preferably added by 830 - 840 **mg** to 100 **Mℓ** of the water-soluble solvent, but not always limited thereto.

In the above method, the hydrophobic bile acid of step 2) can be any bile acid, and preferably 5β-cholanic acid represented by formula 1. The organic solvent herein can be any organic solvent capable of dissolving hydrophobic bile acid and preferably methanol. The bile acid herein is preferably added by 6 - 60 **mg** to 100 **Mℓ** of the organic solvent, but not always limited thereto.

In the above method, the reaction of step 3) is preferably performed with stirring after slowly loading the bile acid solution to the albumin solution, but not always limited thereto. At this time, a catalyst to conjugate bile acid to albumin can be used and the catalyst is exemplified by 1-ethyl-3-(3-dimethyl-aminopropyl) carbodiimide (EDC) and N-hydrosuccinimide (NHS), but not always limited thereto. When EDC is used as a catalyst, the concentration is preferably 0.9 - 1.1 weight part by the concentration of bile acid and when NHS is used, it is used by 1.4 - 1.6 weight part by the weight of EDC, but not always limited thereto. It is preferred to add the catalyst to the reaction mixture of bile acid/albumin. Then, the reaction mixture is stirred for 15 - 30 hours and more preferably 20 - 25 hours, but not always limited thereto. If the reaction time is out of the said range, conjugation of bile acid with albumin is not effectively done.

In step 3), an additional step of including an anticancer agent in the albumin-bile acid conjugate forming self-aggregates of the present invention by loading an anticancer agent solution prepared by dissolving an anticancer agent in an organic solvent to the reaction solution can be included.

An anticancer agent included in the said albumin-bile acid conjugate can be any hydrophobic anticancer agent selected from the group consisting of Docetaxel, cis-platin, camptothecin, paclitaxel, Tamoxifen, Anasterozole, Gleevec, 5-FU, Floxuridine, Leuprolide, Flutamide, Zoledronate, Doxorubicin, Vincristine, Gemcitabine, Streptozocin, Carboplatin, Topotecan, Belotecan, Irinotecan, Vinorelbine, hydroxyurea, Valrubicin, retinoic acid, Methotrexate, Meclorethamine, Chlorambucil, Busulfan, Doxifluridine, Vinblastin, Mitomycin, Prednisone, Testosterone, Mitoxantron, aspirin, salicylates, ibuprofen, naproxen, fenoprofen, indomethacin, phenyltazone, cyclophosphamide, mechlorethamine, dexamethasone, prednisolone, celecoxib, valdecoxib, nimesulide, cortisone and corticosteroid, but not always limited thereto. For a solvent to dissolve an anticancer agent, any solvent capable of dissolving an anticancer agent can be used, but ethyl alcohol is preferred.

The physical method to include an anticancer agent in the albumin-bile acid conjugate of the present invention has inclusion efficiency of 30 - 50%, which is significantly higher than that of the chemical binding method (maximum inclusion efficiency: 10%), suggesting that this method can overcome the limit of the conventional chemical binding method and thereby can extend drug content significantly.

In the above method, the dialysis of step 4) is performed by the conventional method, and the reaction solution excluding non-reacted bile acid is freeze-dried to give the albumin-bile acid conjugate of the present invention.

In step 4), an additional step of conjugating fore infrared ray fluorescent material to the albumin-bile acid conjugate to prepare albumin-bile acid-fore infrared ray fluorescent material conjugate can be included.

Preferably, the fore infrared ray fluorescent material is dissolved in a solvent, which is added to the albumin-bile acid conjugate solution, followed by stirring. Liquid chromatography is performed for separation, followed by freeze-drying to prepare albumin-bile acid-fore infrared ray fluorescent material conjugate, but not always limited thereto.

The fore infrared ray fluorescent material herein is preferably selected from the group consisting of cyanine (Cy), fluorescein, tetramethylrhodamine, BODIPY and Alexa, and Cy5.5 is more preferred, but not always limited thereto.

The solvent used in this invention to dissolve the albumin-bile acid conjugate and the fore infrared ray fluorescent material is preferably PBS, but not always limited thereto. The preferable concentration of the fore infrared ray fluorescent material is 2 - 5 weight part by the weight of the albumin-bile acid conjugate, but not always limited thereto.

The present invention also provides use of the said albumin-bile acid conjugate in the manufacture of a drug delivery carrier.

The drug herein is preferably an anticancer agent, and the anticancer agent can be any hydrophobic anticancer agent that can be included physically in albumin-bile acid conjugate.

The albumin-bile acid conjugate of the present invention is composed of hydrophilic albumin and hydrophobic bile acid, so that it can form self-aggregates in aqueous solution and favors inclusion of a hydrophobic anticancer agent in the conjugate and has excellent tumor targeting capacity, so that it can be effectively used as a component of an anticancer agent delivery carrier.

The present invention also provides a method for treating cancer comprising the following steps:
1) mixing an anticancer agent with the said albumin-bile acid conjugate, and forming an albumin-bile acid nanoparticle containing the anticancer agent thereby; and
2) administering the nanoparticle to a subject.

The albumin-bile acid conjugate of the present invention is composed of hydrophilic albumin and hydrophobic bile acid, so that it can form self-aggregates in aqueous solution and favors inclusion of a hydrophobic anticancer agent in the conjugate and has excellent tumor targeting capacity, so that it can be effectively used as a component of an anticancer agent delivery carrier.

In general, self-aggregate is a round shaped aggregate formed by congregation of amphiphilic molecules having both hydrophilic group and hydrophobic group in aqueous solution. Hydrophilic group are gathered outside of the round aggregate, while hydrophobic groups are gathered inside of the aggregate (Adv. Drug Deliv. Rev., 1996, 21, 107). So, it has been widely used as an efficient drug delivery system for various hydrophobic anticancer agents. Such drug delivery system using the amphiphilic molecules capable of forming self-aggregates has advantages of satisfactory target selectivity, significant low toxicity in normal cells and continuous drug releasing *in vivo* for a long period of time, so that it can be used in study of a novel treatment method for cancer as a promising drug delivery system.

The albumin-bile acid conjugate in which a hydrophobic anticancer agent is included has higher tumor selectivity than the conventional low-molecular anticancer agent owing to EPR (enhanced permeability and retention) effect, so that this formulation can be accumulated in tumor tissues at a high concentration, resulting in improved anticancer activity. This conjugate can form micelle-like round shaped self-aggregates in aqueous solution by the conjugation of hydrophobic bile acid to hydrophilic albumin used as a main chain. Therefore, as an anticancer agent delivery carrier, it is target-oriented and has high anticancer effect by releasing the drug constantly, making it a useful candidate for the treatment of cancer.

The present invention also provides a contrast agent for cancer diagnosis comprising albumin-bile acid-fore infrared ray fluorescent material conjugate composed of the said albumin-bile acid conjugate which is composed of hydrophilic albumin and hydrophobic bile acid so that it can form self-aggregates in aqueous solution and fore infrared ray fluorescent material.

The fore infrared ray fluorescent material herein is selected from the group consisting of cyanine (Cy), fluorescein, tetramethylrhodamine, BODIPY and Alexa, and more preferably it is Cy5.5, but not always limited thereto.

The said contrast agent for cancer diagnosis can additionally include radio isotope, quantum dot and MRI contrast agent, but not always thereto and can contain any material which can be included in the conventional contrast agent.

The present invention also provides a method for diagnosing cancer comprising the following steps:
1) administering the said contrast agent to a subject; and
2) imaging of tumor tissues after irradiating fore infrared ray.

The albumin-bile acid conjugate of the present invention forms macro nano-particles, compared with pure albumin, suggesting that it has high tumor selectivity by EPR effect, so that it can bring high accumulation efficiency in tumor tissues and enables longer stay *in vivo* than the conventional albumin. Fore infrared ray fluorescence is generated by fore infrared ray fluorescent filter, and *in vivo* imaging can be performed by using fore infrared ray, PET/SPECT and CCD camera. By fore infrared ray irradiation, a small volume of tumor in the early stage which is about 1 mm in size can be diagnosed. Therefore, it can be effectively used as a novel contrast agent for cancer diagnosis.

In addition, the present invention provides use of the said albumin-bile acid-fore infrared ray fluorescent material conjugate in the manufacture of a contrast agent for cancer diagnosis.

The albumin-bile acid conjugate of the present invention forms macro nano-particles, compared with pure albumin, suggesting that it has high tumor selectivity by EPR effect, so that it can bring high accumulation efficiency in tumor tissues and enables longer stay *in vivo* than the conventional albumin. Fore infrared ray fluorescence is generated by fore infrared ray fluorescent filter, and *in vivo* imaging can be performed by using fore infrared ray, PET/SPECT and CCD camera. By fore infrared ray irradiation, a small volume of tumor in the early stage which is about 1 mm in size can be diagnosed. Therefore, it can be effectively used as a component for the production of a contrast agent for cancer diagnosis.

Practical and presently preferred embodiments of the present invention are illustrative as shown in the following Examples, Experimental Examples and Manufacturing Examples.

However, it will be appreciated that those skilled in the art, on consideration of this disclosure, may make modifications and improvements within the spirit and scope of the present invention.

### Example 1: Preparation of albumin-bile acid (5β-cholanic acid)-Cy5.5 conjugate

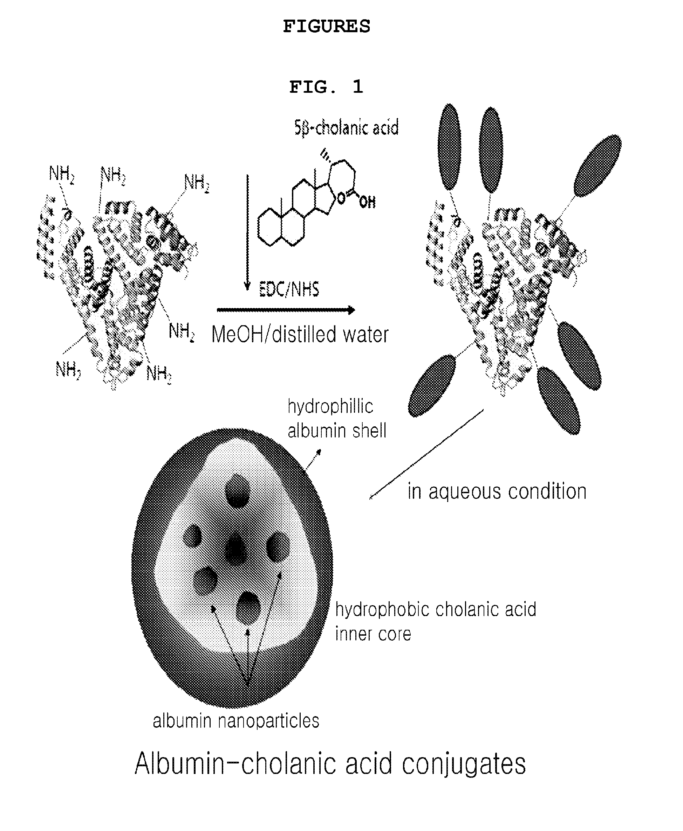

500 mg of albumin was dissolved in 60 ml of water, to which 90 ml of methanol was added to prepare albumin solution. Bile acid, 5β-cholanic acid represented by formula 1, was added to 100 ml of methanol at the mass ratio of 10-100% (6 mg-60 mg) by 10% each. As a catalyst for chemical reaction, 1-ethyl-3-(3-dimethyl-aminopropyl) carbodiimide (EDC) and N-hydrosuccinimide (NHS) were dissolved in 50 ml of methanol at the concentration of 1.5 times the molar concentration of bile acid, which was added to the reaction solution, followed by stirring at room temperature for 24 hours. The reaction solution was dialyzed for 2 days to eliminate non-reacted 5β-cholanic acid, followed by freeze-drying. As a result, the albumin-5β-cholanic acid of the present invention was prepared (Figure 1).

To measure the *in vivo* tumor targeting capacity, 5 mg of albumin-bile acid conjugate was dissolved in 0.8 ml of PBS (phosphate buffer saline) and 2 weight% of Cy5.5 monoreactive hydroxysuccimimide ester (Cy-5.5-NHS) was dissolved in 0.2 ml of PBS. While stirring the albumin-bile acid solution, the Cy-5.5-NHS solution was loaded. The mixed solution was covered by aluminum foil to block the light, followed by stirring at room temperature for 1 hour. The reactant was separated by FPLC(fast protein liquid chromatography) [moving rate: 0.7 ml/min, fraction eluent: 1ml, 675 nm (cy), 280 nm (albumin) wavelength], followed by freeze-drying to give albumin-bile acid-Cy5.5 conjugate.

In the above reaction, albumin-bile conjugate in which bile acid was conjugated to albumin more than 50% was not dispersed well and precipitated because of excessive hydrophobicity, so that this conjugate was excluded in the following examples.

### Example 2: Analysis of albumin-bile acid-Cy5.5 conjugate nano-particles

To observe the optical properties of the albumin-bile acid-Cy5.5 conjugate prepared in Example 1, 1 mg of the albumin-bile acid-Cy5.5 conjugate was dissolved in 1 ml of PBS, followed by analysis using fore infrared ray fluorescence spectrophotometer (F-7000 model, HITACHI high technologies corporation).

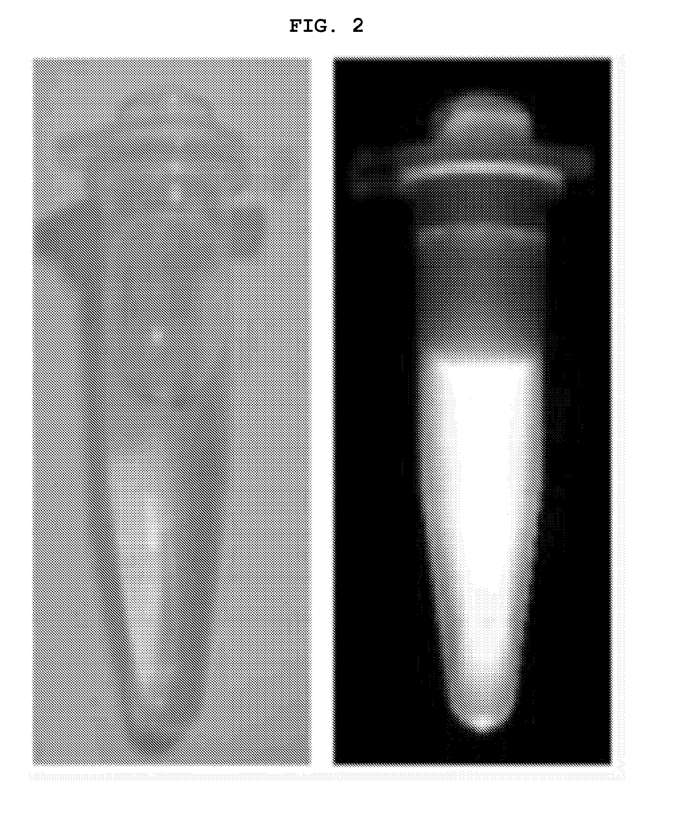

As a result, as shown in Figure 2, the albumin-bile acid-Cy5.5 conjugate of the present invention generated fore infrared ray fluorescence from fore infrared ray fluorescence filter (Figure 2).

To observe the change of albumin nano-particle size according to the increase of 5β-cholanic acid content in the albumin-bile acid-Cy5.5 conjugate, 1 mg of the albumin-bile acid-Cy5.5 conjugate was dissolved in 1 ml of PBS, followed by measurement of particle size of each conjugate using DLS (dynamic light scattering, 127 model, Brook haven instruments corporation BIC).

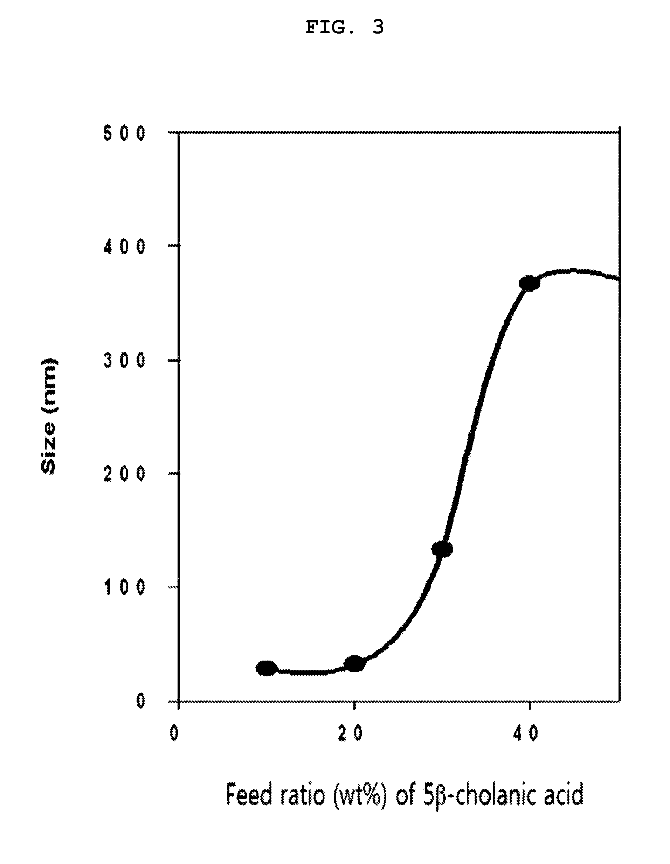

As a result, as shown in Figure 3, when mass ratio of 5β-cholanic acid to albumin was 10%, particle size was approximately 30 nm in diameter. Until the mass ratio reached 40%, the size of nano-particle kept increasing. When the mass ratio was 40%, the particle size was approximately 400 nm in diameter. However, when the mass ratio was raised from 40%, the size of nano-particle was not increased any more (Figure 3). Therefore, as the concentration of bile acid in albumin was increased, nano-particles of 30 nm - 400 nm in size were produced.

To observe the shape of particle of the albumin-bile acid-Cy5.5 conjugate in aqueous solution, transmission electron microscopy (TEM, PHILIPS) was used.

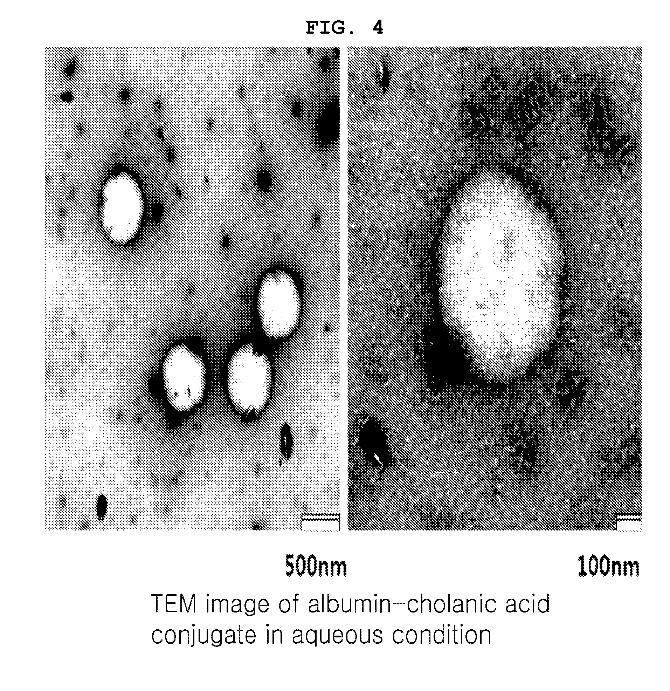

As a result, as shown in Figure 4, particles of the albumin-bile acid-Cy5.5 conjugate of the present invention were very regular in aqueous solution (Figure 4).

### Example 3: Drug inclusion efficiency of albumin-bile acid-Cy5.5 conjugate

To investigate the hydrophobic anticancer agent inclusion efficiency of the albumin-bile acid-Cy5.5 conjugate prepared in Example 1, the present inventors performed following experiment. 5 mg of albumin-bile acid-Cy5.5 was dissolved in 5 ml of PBS. 0.5 mg (10%) of Taxsol was dissolved in 0.5 ml of methanol (total volume: up to 10%). The above two solutions were mixed and fully stirred, followed by sonication for 5 minutes, stirring for 3 hours, and dialysis (MWCO 12000-14000). Centrifugation was performed at 2000 rpm for 15 minutes and then the supernatant was freeze-dried. 1 mg of inclusion body was dissolved in 1 ml of methanol, followed by sonication for 10 minutes and stirring for 12 hours. The solution was centrifuged at 10,000 rpm for 15 minutes. The supernatant was filtered with 0.45 µ**m** filter, followed by quantification by HPLC.

As a result, it was confirmed that the anticancer agent inclusion efficiency of the albumin-bile acid-Cy5.5 conjugate of the present invention was 30-50%.

### Example 4: Evaluation of drug accumulation effect of albumin-bile acid-Cy5.5 conjugate in tumor tissues

To investigate the drug accumulation effect of the albumin-bile acid-Cy5.5 conjugate prepared in Example 1 in tumor tissues, the present inventors performed following experiment. 100 µℓ of each albumin (control group) and albumin-bile acid-Cy5.5 conjugate (mass ratio of bile acid to albumin was 10 wt% and 20wt%) nano-particle solution (5 mg/kg) (experimental group) was intravenously injected into mice injected with skin cancer cells (SCC-7 cell, balb-c nude mouse, Central Lab. Animal Inc., Korea). 1, 6, 12, 24 and 48hours later, fore infrared ray was irradiated and images of tumor tissue of up to 1 cm were obtained by using fore infrared ray fluorescence spectrophotometer (F-7000 model, HITACHI high technologies corporation).

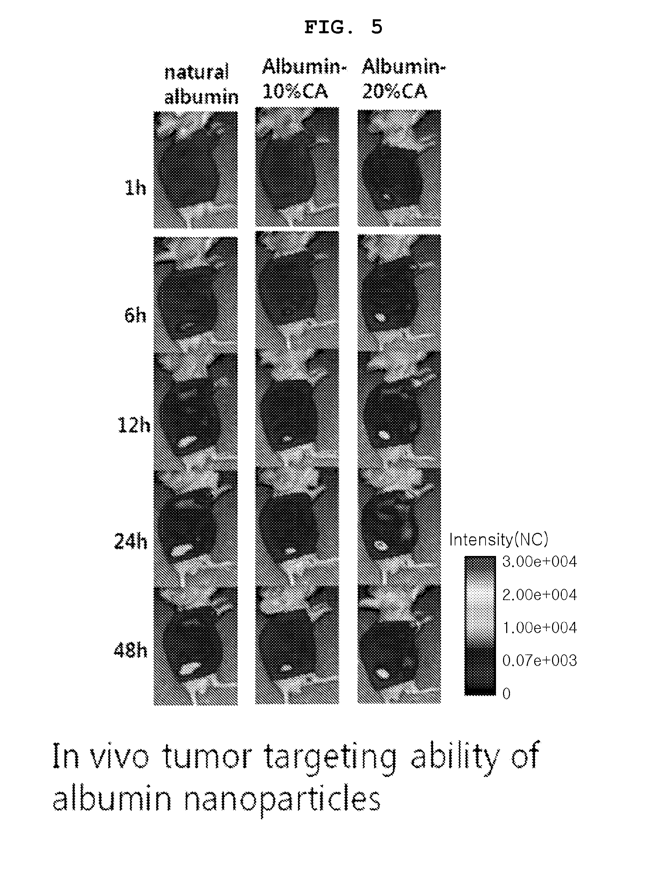

As a result, the albumin-bile acid-Cy5.5 conjugate in which 20 w% of bile acid was conjugated was confirmed to be accumulated in tumor tissues significantly, compared with the pure albumin (Figure 5). The above result suggests that the albumin-bile acid-Cy5.5 conjugate of the present invention demonstrated significantly improved accumulation efficiency in tumor tissues, so that it can be effectively applied in the development of an anticancer agent carrier and diagnosis of cancer based on its tumor targeting capacity.

### Example 5: Investigation of anticancer effect of albumin-bile acid-Cy5.5 conjugate containing a hydrophobic anticancer agent

To investigate the anticancer effect of the albumin-bile acid-Cy5.5 conjugate containing Taxsol prepared in Example 3, the present inventors performed following experiment. 20 **mg/kg** or 50 **mg/kg** of the albumin-bile acid-Cy5.5 conjugate containing Taxsol of the present invention was administered to the melanoma mice (C57BL/6) transplanted with skin cancer cells (B16F10) for 21 days. Then, the test animals were weighed and the sizes of tumors were measured. At this time, Cremophor EL (polyethoxylated castor oil derivatives, BASF) containing Taxsol was used for the control group and saline was used for the negative control.

The size of tumor in the negative control mouse administered with saline was increased over the time. But, the size of tumor in the test animal administered with Cremophor EL containing Taxsol or the albumin-bile acid-Cy5.5 conjugate containing Taxsol of the present invention was not changed for about 9 days. The size of tumor in the mouse administered with albumin-bile acid-Cy5.5 conjugate containing Taxsol was decreased as the concentration was raised. Observation of weight changes showed consistent results. So, the albumin-bile acid-Cy5.5 conjugate of the present invention was confirmed to be effectively used as an anticancer agent delivery carrier.

### INDUSTRIAL APPLICABILITY

The albumin-bile acid conjugate of the present invention can include a hydrophobic anticancer agent and be used as a nano-particle drug delivery carrier. It also can be used as a contrast agent for cancer diagnosis by being conjugated with fore infrared ray fluorescent material. So, this conjugate can be further used for the development of a novel nano-particle contrast agent and a nano-particle drug delivery system for the diagnosis and treatment of cancer.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. An albumin-bile acid conjugate for drug delivery, which is composed of hydrophilic albumin and hydrophobic bile acid so that it forms self-aggregate in aqueous solution.

2. The albumin-bile acid conjugate for drug delivery according to claim 1, wherein amino group (-NH2) of lysine group of albumin and carboxyl group (-COOH) of bile acid are conjugated by peptide bond.

3. The albumin-bile acid conjugate for drug delivery according to claims 1 or 2, wherein the bile acid is 5β-cholanic acid.

4. The albumin-bile acid conjugate for drug delivery according to any one of claims 1 to 3, wherein the size of particle of the albumin-bile acid conjugate is increased in proportion to the weight part of bile acid and is 30-400 nm in diameter.

5. The albumin-bile acid conjugate for drug delivery according to any one of claims 1 to 4, wherein the drug is a hydrophobic anticancer agent.

6. The albumin-bile acid conjugate for drug delivery according to any one of claims 1 to 5, wherein the albumin-bile acid conjugate is additionally conjugated with fore infrared ray fluorescent material.

7. The albumin-bile acid conjugate for drug delivery according to claim 5, wherein the hydrophobic anticancer agent is additionally included in the albumin-bile acid conjugate.

8. A method for preparing the albumin-bile acid conjugate of any one of claims 1 to 7 comprising the following steps:
1) preparing albumin solution by dissolving hydrophilic albumin in a water-soluble solvent;
2) preparing bile acid solution by dissolving hydrophobic bile acid in an organic solvent;
3) preparing reaction solution by mixing the albumin solution of step 1) and the bile acid solution of step 2) and stirring thereof; and
4) dialyzing the reaction solution of step 3) and eliminating non-reacted bile acid, followed by freeze-drying thereof.

9. The method for preparing the albumin-bile acid conjugate according to claim 8, wherein the bile acid of step 2) is 5β-cholanic acid.

10. Use of the albumin-bile acid conjugate of any one of claims 1 to 7 in the manufacture of a drug delivery carrier.

11. A method for treating cancer comprising the following steps:
1) mixing an anticancer agent with the albumin-bile acid conjugate of any one of claims 1 to 7 and forming an albumin-bile acid nanoparticle containing the anticancer agent thereby; and
2) administering the nanoparticle to a subject.

12. A contrast agent for cancer diagnosis comprising albumin-bile acid-fore infrared ray fluorescent material conjugate composed of the albumin-bile acid conjugate of any one of claims 1 to 7 and fore infrared ray fluorescent material.

13. The contrast agent for cancer diagnosis according to claim 12, wherein the agent additionally includes radio isotope, quantum dot or MRI contrast agent.

14. A method for diagnosing cancer comprising the following steps:
1) administering the contrast agent of claims 12 or 13 to a subject; and
2) imaging tumor tissues after irradiating fore infrared ray.

15. Use of the albumin-bile acid-fore infrared ray fluorescent material conjugate of claims 12 or 13 in the manufacture of a contrast agent for cancer diagnosis.
